# EUROPEAN PATENT SPECIFICATION

(11) **EP 3 148 415 B1**
(45) Date of publication and mention of the grant of the patent: **23.10.2019**
(21) Application number: 15799317.1
(22) Date of filing: 28.05.2015
(51) Int. Cl.: A61B 5/00, A61B 5/145, A61B 5/1477, G01N 33/487

(54) **VERTICAL-FLOW ELECTRONIC BIO-CHEMICAL SENSING DEVICES**
ELEKTRONISCHE BIOCHEMISCHE VERTIKALFLUSSMESSVORRICHTUNGEN
DISPOSITIFS DE DÉTECTION BIOCHIMIQUES ÉLECTRONIQUES À ÉCOULEMENT VERTICAL

(30) Priority: 28.05.2014 US 201462003715 P; 28.05.2014 US 201462003692 P
(43) Date of publication of application: 05.04.2017
(60) Divisional application: 19188927.8
(73) Proprietor: University of Cincinnati, Cincinnati, OH 45206 (US)
(72) Inventor: HEIKENFELD, Jason C., Cincinnati, OH 45230 (US)
(74) Representative: Findlay, Alice Rosemary
(86) International application number: PCT/US2015/032830
(87) International publication number: WO 2015/184065

(56) References cited:
- EP-A1- 0 453 283
- US-A1- 2008 045 816
- US-A1- 2010 044 224
- US-A1- 2011 196 283
- US-A1- 2015 112 164
- US-A1- 2015 112 165
- US-B1- 6 269 265
- US-B2- 7 813 780
- US-B2- 7 959 791

## Description

### BACKGROUND OF THE INVENTION

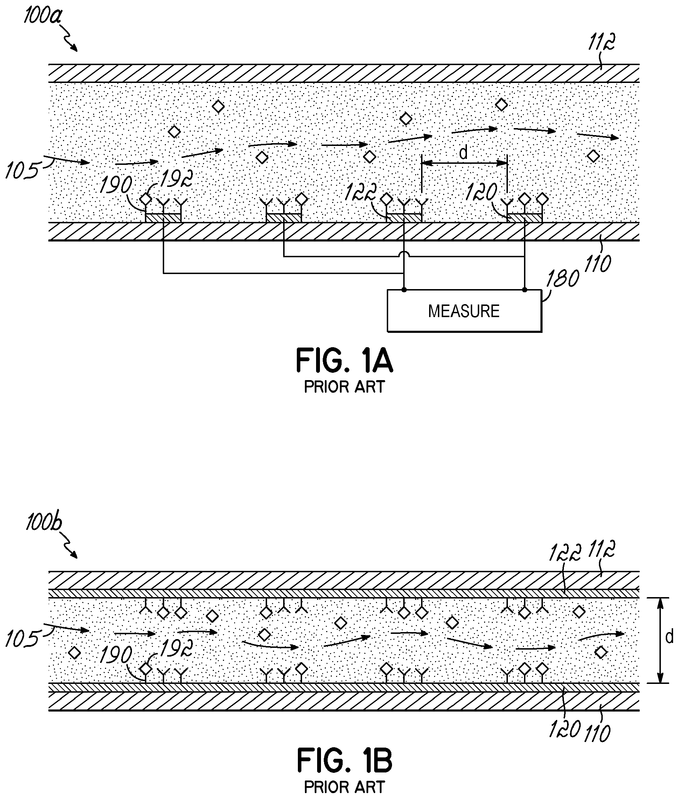

Numerous technologies have been developed to detect chemical solutes and various compounds (e.g., analytes) in the body. Many of these techniques measure impedance or charge transfer, or use techniques which benefit from closely spaced interdigitated electrodes. Fig. 1A shows a conventional impedance measuring, or impedance spectroscopy, device 100a used to detect chemical solutes and/or various compounds in the body. Sensing device 100a includes a first substrate 110, which may be made of glass or plastic for example, that carriers pairs of interdigitated electrodes 120, 122 separated by a spacing or pitch ("d"). The pitch ("d") is typically made as small as possible, often only a few micrometers, to maximize the performance of the sensing device 100a. This creates challenges when integrating such technology with simple low-cost printed electronics or standard circuit board technology, which cannot achieve such finely patterned features. The sensing device 100a includes probes or biorecognition elements 190, which selectively capture analytes or biomarkers 192 in a horizontal flow of analytes 105. The horizontal flow of analytes 105 can be through a porous solid, gel, liquid gas, or other material suitable for transporting or permitting movement or transport of the analytes 192. Any method of transport for analytes is possible, including by diffusion, by advective flow in a fluid or gas, by iontophoresis, by electro-osmosis, or by other suitable techniques. As analytes 192 are captured onto probes 190, the measurement circuit 180 is able to detect charge transfer, changes in impedance, or other electrically measureable changes known by those skilled in the art that indicate the presence of analyte 192. These changes can even be used in some cases to measure the concentration of analyte 192 in flow 105. Two of several challenges of device 100a are the complexity of fabrication of the interdigitated electrodes 120, 122 and the low amount of diffusion or transport of analytes in flow 105 down to the probes 190, especially so at low analyte concentrations.

With reference to Fig. 1B, a prior art sensing device 100b is shown where the upper and lower walls, or any walls, of a microfluidic channel carry the sensing electrodes 120, 122. The channel and spacing between electrodes 120, 122 is defined by the spacing ("d") between substrates 110, 112. All like numeral features provided and are similar to those described for Fig. 1. The device 100b allows a simple and reliable way to achieve closely spaced electrodes, which benefits numerous sensing modalities, and the flow of analytes 105 is always close to the probes 190 such that even at low concentrations of analytes 192 the analytes can more easily diffuse or transport to the probes 190. However, the configuration shown for device 100b will dramatically reduce the ease or rate of transport of flow of analytes 105, and in some cases, will be difficult to integrate with various complete bio or chemical sensor device designs.

Such devices require a sample to be introduced to a sensor surface or material. Often, the preferred requirements of sample introduction and of the sensor configuration are conflicting in one or more aspect, such that the performance of the device is reduced in one or more aspect. Furthermore, in many cases the fluid volume is very small, presenting unique challenges to the sensor configuration. In addition, the sensors themselves often require complicated configurations such as spaced electrodes to achieve greater performance.

EP 0453283 discloses a fluid sensor which comprises a substrate or a porous material for the passage of the interstitial fluid therethrough. At least two electrodes are provided. At least one of the electrodes has one side mounted on the substrate. The electrode is also of a porous material and permits the passage of the interstitial fluid from one side through to the second side which is generally opposite the one side. A layer of chemical is on the second side. The layer of chemical comprises a chemical for reaction with one component of the fluid, mixed in a mediating agent. The electrodes generate an electrical signal upon the detection of the reaction of the one component of the fluid with the chemical.

### SUMMARY OF THE INVENTION

The invention is defined by the claims.

### BRIEF DESCRIPTION OF THE DRAWINGS

The objects and advantages of the present invention will be further appreciated in light of the following detailed descriptions and drawings in which:
Fig. 1A is a schematic of a conventional sensing device.
Fig. 1B is a cross-sectional view of a conventional sensing device.
Fig. 2A is a cross-sectional view of a sensing device according to one embodiment of the present invention placed on skin.
Fig. 2B is a cross-sectional view of a sensing device according to one embodiment of the present invention incorporated into a lateral flow paper-microfluidic device.
Fig. 3A is a cross-sectional view of a sensing device according to one embodiment of the present invention.
Fig. 3B is a cross-sectional view of a portion of the sensing device of Fig. 3A showing a porous configuration.
Fig. 3C is a cross-sectional view of the sensing device of Fig. 3A showing an analyte flow.
Figs. 4A-C are cross-sectional views of sensing devices according to various embodiments of the present invention having different configurations.
Fig. 5A is a cross-sectional view of a sensing device according to one embodiment of the present invention placed on skin.
Fig. 5B is a cross-sectional view of the sensing device of Fig. 5A.
Fig. 5C is a cross-sectional view of a sensing device according to one embodiment of the present invention.
Fig. 6 is a cross-sectional view of a sensing device according to one embodiment of the present invention placed on skin.

### DETAILED DESCRIPTION

The embodiments of the present invention described below are described primarily in terms of analytes transported in a fluid and applications such as wearable biomarker sensing device. However, the present invention is not so limited in application or functionality. The present invention applies to any electronic sensing modality that would benefit in at least one performance, cost or other valued metric for chemical or biomarker (i.e. analyte) sensing. The embodiments of the present invention discussed below are illustrated primarily focusing on the sensor devices themselves. For purposes of convenience, the descriptions may not include illustration or description of additional features or system-level components, software, batteries, or other components that may be needed for proper function that are obvious to those skilled in the art.

Features of the present invention apply to transport or flow of analytes through any material, including a porous solid, gel, liquid, gas, or other material suitable for transporting or permitting movement or transport of the analytes. Embodiments of the present invention may include any method of transport for analytes including, for example, diffusion, advective flow in a fluid or gas, iontophoresis, electro-osmosis, or other suitable techniques. The present invention contemplates any sensing modality or variation of embodiments of the present invention that benefit in performance, cost, convenience, sampling interval, longevity, limit of detection, specificity, ease of integration of additional features, ease of integration within a device, or any other aspect of bio or chemical sensing which can benefit from the embodiments of the present invention. Embodiments of the present invention will generally exhibit signal-to-noise advantages in sensors where a charge transfer between the electrode and solution occurs, including amperometric and impedance type sensing modalities and other sensing modalities which are known to benefit from use of interdigitated electrodes. Embodiments of the present invention will generally also allow sensors to stabilize more quickly, as the diffusive path length to the sensor surface is generally reduced.

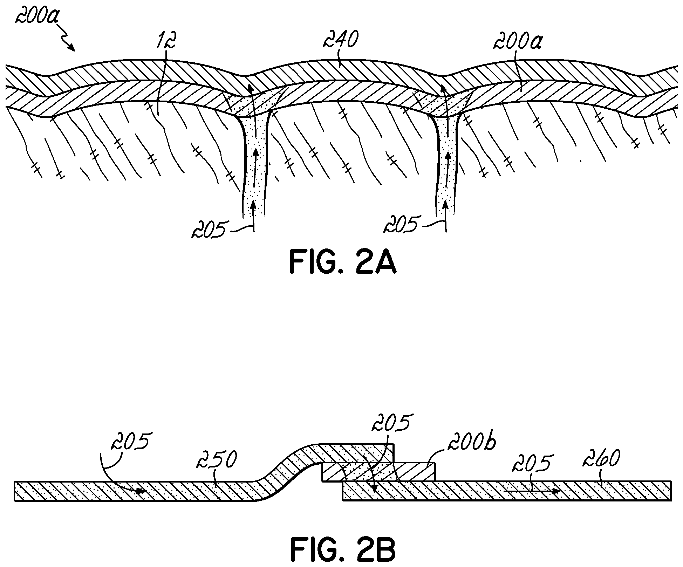

Embodiments of the present invention may be advantageously integrated in a variety of applications, some of which are illustrated in Figs. 2A-2C. With reference to Fig. 2A, a porous sensing device 200a is shown which is laminated onto or placed adjacent to skin or tissue 12. Sensing device 200a uses absorbent or evaporative element 240 to transport sweat fluid or sweat analytes vertically in flow 205 from skin 12 through the porous sensing device 200a and onto element 240. Sensing device 200a is vertically porous and thin such that flow 205 occurs easily and quickly through sensing device 200a. Embodiments discussed below include further details on the porosity of a sensing device. In this embodiment, there is very little dead volume of fluid created, and very little fluid is needed to allow sensing device 200a to function properly. Further, additional fluidic controls are not needed.

Fig. 2B shows a sensing device 200b incorporated into a lateral flow paper-microfluidic device. The lateral flow paper-microfluidic device includes a paper strip 250, which introduces fluid flow 205 through sensing device 200b, and a paper strip 260. The paper strip 250 introduces fluid flow 205 through the sensing device 200b, which is porous. The fluid flow 205 then passes to the paper strip 260, which carries fluid flow 205 away from sensing device 200b. In various embodiments, sensing devices 200b may be incorporated into lateral flow assay devices or simple flex electronic or wearable sensor devices, which must be thin and not made from rigid channel microfluidics.

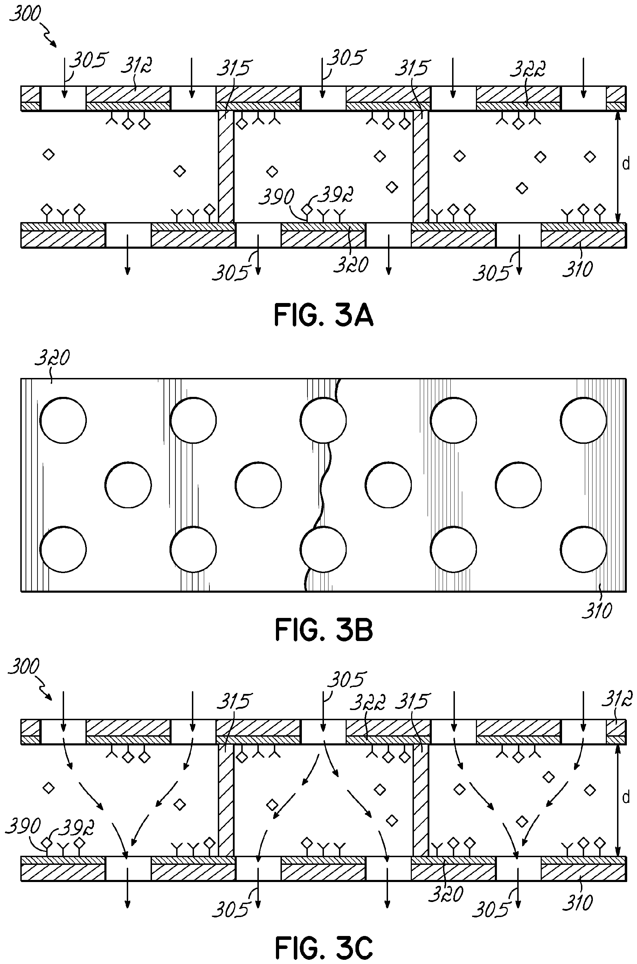

With reference to Fig. 3A, a sensing device 300 includes substrates 310, 312 and spacer materials 315. Substrates 310, 312 may be, for example, porous membranes, porous films, meshes, or any other suitable porous material, such as commercial track-etch membranes. In the illustrated embodiment, substrates 310, 312 are coated with optional electrodes 320 and 322 and probes 390. Fig. 3B provides a top view of pores in substrate 310 and electrode 320. In embodiments where substrates 310 and 312 are electrically conductive themselves, a sensing device may exclude the optional electrodes 320, 322. In one embodiment, substrates 310, 312 are porous metal films or porous carbon fiber films and thereby provide electrically conductive surfaces. The porosity of the device 300 allows an analyte flow 305, which is shown in Fig. 3C in greater detail. Although not shown, substrate 310 has a first inner surface and a first outer surface. Further, substrate 312 includes a second inner surface and a second outer surface. Substrates 310, 312 overlie each other and are spaced a distance ("d") apart, which may be roughly the distance between the first and second inner surfaces. Substrates 310, 312 may be spaced apart using spacer materials 315. The spacer materials 315 are porous and may be, for example, thin adhesives, spacer balls as are used in LCD displays, a photoresist, or an adhesive coated textile or mesh, among other materials. The spacers 315 are typically easy to fabricate, even down to only several micrometers across, using techniques known by those skilled in the art, and can regulate the spacing between the electrodes such that the impedance is adequately regular, predictable, and homogeneous. Sensing device 300 includes a variety of unique aspects and advantages. For instance, sensing device 300 is easily manufactured with a small electrode spacing ("d") and may have a thin and flexible construction. Sensing device 300 may be manufactured using low-cost fabrication methods such as roll-to-roll fabrication. Further, sensing device 300 can offer little resistance to fluid flow 305, and analytes 392 are brought into close proximity to probes 390 such that high performance sensing can be achieved for analytes at low concentrations and/or with low diffusivity. Another exemplary advantage is that the alignment of substrates 310, 312 is not required. Those skilled in the art may recognize further advantages. In another aspect of the present invention, the sensing device 300 could be used to provide detection by measurement of impedance, potential, current, frequency response, or other known methods of bio or chemical sensing technologies.

A variety of analyte probes or electrical sensing methods are useful in embodiments of the present invention. By way of example, a probe or electrical sensing method may be an aptamer, redox couples, an antibody layer, an enzyme layer, or an ionophore membrane. Further, a surface that is selective in some way for sensing without a specific probe layer (e.g., stripping voltammetry) may be used. Generally, any surface that provides an electrical response to the presence of an analyte is adequate for use in embodiments of the present invention. Even surfaces that utilize an insulator on an electrically conductive surface, such as electrical capacitance or field-effect type sensors, are included since they also have an electrically conductive surface, and hence have an electrical response (be it direct or indirect) to the presence of said analyte.

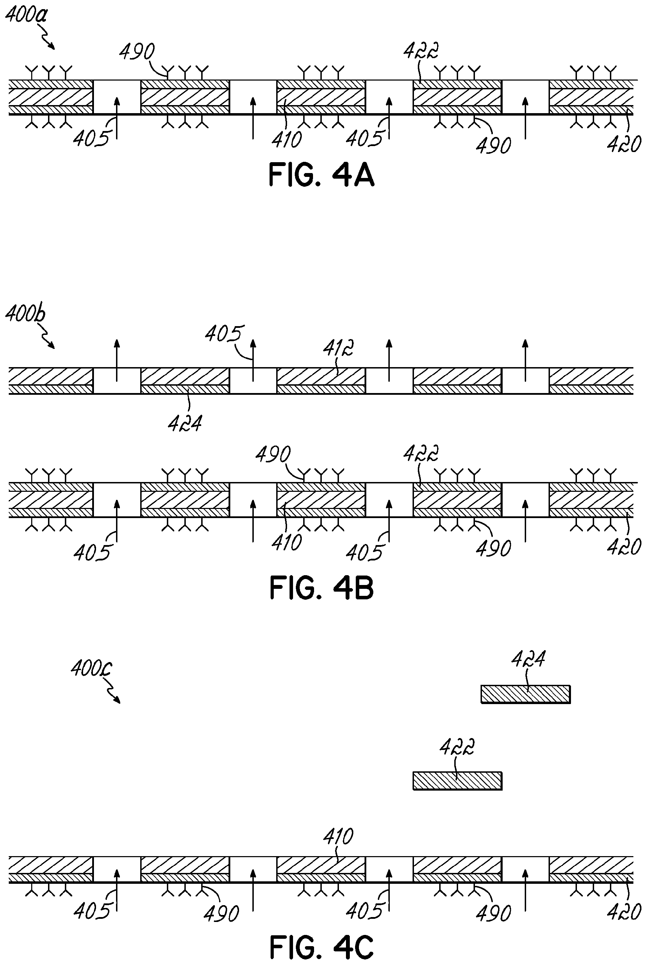

Figs. 4A-4C illustrate various embodiments of the present invention having a sensing device including one or more substrates, electrodes, and probes in a variety of configurations. With reference to Fig. 4A, at least a portion of a sensing device 400a is shown, consisting of a porous substrate 410, electrodes 420, 422, and a coating or layer of probes for sensing 490. Similar to device 300a, device 400a includes advantageous the aspects of electrodes 420, 422 being in close proximity, the ease of the analyte flow 405, and the close proximity of the analytes (not shown) with probes 490. In one embodiment, electrodes 420, 422 may also coat the sides of the pores (not shown) in the substrate 410. Device 400a could be fabricated using several techniques, including angular vacuum metal deposition of a track-etch membrane with high-aspect ratio pores.

With reference to Fig. 4B, a portion of a sensing device 400b is shown. Sensing device 400b includes elements of device 400a and further includes an additional electrode 424 and substrate 412. The location of the probe layer 490 may be determined based on a variety of considerations. For example, the location of the probe layer 490 in device 400b may have been determined because, in this embodiment, the probe layer 490 is easier to manufacture on the external surfaces of the substrate 410 of the device 400b. Those skilled in the art will recognize that a plurality of electrodes or arrangements are possible. Some sensing modalities require at least three electrodes: a reference electrode, a working electrode, and a counter electrode. In this regard, electrodes of device 400b could be any of these exemplary types of electrodes. Various embodiments of the present invention may include electrodes with or without probe layer 490.

With reference to Fig. 4C, a device 400C includes an electrode 420 on a porous substrate 410, and electrodes 422, 424 spaced apart from the porous substrate 410. Alternatively, in one embodiment, electrode 420 may also be porous. Porous electrodes may be, for example, a thin metal film that is porous or a fine metal wire mesh or a porous layer of carbon nanotubes. Utilizing porous electrodes may allow for a simpler manufacturing process.

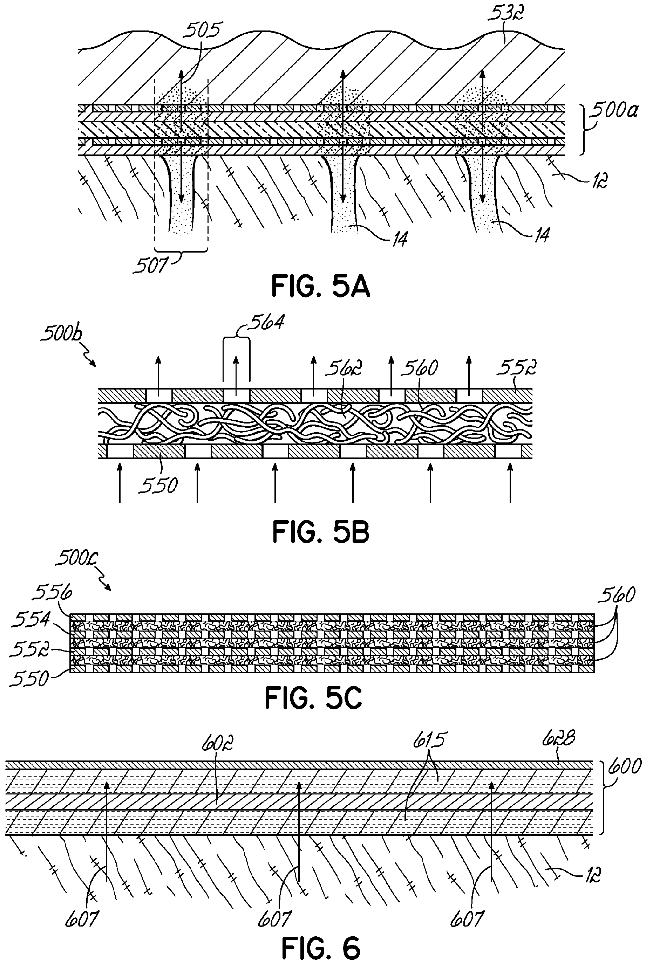

With reference to Fig. 5A, a further embodiment of the present invention is shown as sensing device 500a is pressed onto skin 12. In another embodiment, sensing device 500a could be adhered to skin 12 with an adhesive (not shown). Sensing device 500a includes a wicking material 532 to collect sweat. The device 500a is able to confine fluid from sweat ducts 14 to horizontally confined locations in device 500a, for example as indicated by lines 507. This same configuration therefore confines analyte flow 505. The device 500a may further include elements for stimulating sweat by, for example, iontophoresis. In one embodiment where the device includes a sweat stimulant, such as pilocarpine, the flow 505 could also confine the iontophoresis of the sweat stimulant into just the sweat ducts 14 or dominantly in areas near the sweat ducts 14. Device 500a could achieve confinement of the flow 505 in several ways. In one embodiment, the space between two substrates by which device 500a is fabricated is sealed together with spacers that form a lattice of horizontally closed cells (e.g., a honeycomb lattice). In this embodiment, the spacers would form the sidewalls for each closed cell. In an advantageous aspect of the present invention, confining the horizontal flow reduces the overall sweat volume. Reducing the total volume of sweat needed to flow through or to the sensors may improve the sampling interval and/or may allow sensing at very low sweat rates. Some sensor types, such as impedance, amperometric, or others, require fluid to make an electrical contact between two electrodes. In embodiments where the sensor requires fluid to make electrical contact between two electrodes and where the sensor has a configuration similar to device 500a, the sensed signal would be limited to where fresh sweat is flowing. In the other areas of the device where no sweat is present, the background signal would be very low due to much higher electrical impedance.

With reference to Fig. 5B, device 500b is shown as an exemplary configuration of device 500a of Fig. 5A. Device 500b includes porous layers 550, 552 and porous material 560. Layers 550, 552 include a substrate component and an electrode component, which could be an electrically conductive surface on the substrate component. Porous material 560 may act as a spacer layer. Sweat will flow vertically through device 500b, but the flow of the sweat horizontally through device 500b is restricted. The horizontal confinement of fluid is achieved by Laplace pressure and the fluid path (not shown) must be pressure permeated by pressure of sweat. This can be achieved by implementing materials for porous layers 550, 552 such that they are non-wicking to a fluid. In various embodiments, porous layers 550, 552 may be hydrophobic or only slightly hydrophilic. In one embodiment where porous layers 550, 552 are made of a polymer, and porous material 560 is made of a polymer with the same or similar wetting contact angle as the polymer of layers 550, 552, then a smaller pore diameter 562 compared to the pore diameter 564 would confine fluid flowing under pressure against device 500b, which would permeate device 500b substantially vertically.

With reference to Fig. 5C, one embodiment of the present invention includes porous layers 550, 552, 554, 556 and spacer layers 560 in a stacked configuration. In such an embodiment, numerous different types of sensors could be integrated in a single device and benefit from one or more advantages of the present invention.

With reference to Fig. 6, an embodiment of the present invention is shown which utilizes a device 600 including layers of hydrogels 615, an electrode 628, and porous sensor 602, which may be similar to one or more constructions according to embodiments of the present invention discussed above. Electrode 628 may provide an electric field to extract charged solutes or biomarkers by iontophoresis or indirectly by electro-osmotic flow of charged solutes, which drag non-charged solutes along with them. In this regard, device 600 is capable of sensing biomarkers or solutes extracted by electro-osmosis or reverse iontophoresis. Device 600 has a flow of analytes 607 through layers of hydrogels 615 and porous sensor 602. Porous sensor 602 may be constructed according to one or more aspects of the present invention. In one embodiment, for example, porous sensor 602 may be similar to device 300. This is an improvement over previous applications of electro-osmosis and iontophoresis that generally present analyte flow challenges in quickly drawing solutes to the sensor surface and also to the driving electrode for iontophoresis. An additional challenge is also that solutes will build up or concentrate at the electrode driving the iontophoresis, which would contaminate the sensing of new solutes and/or could damage the sensor over time. In the device 600, such concentration of the solutes is not at the porous sensor 602 but instead at the electrode 628, which is further away and is utilized to drive the flow of at least one solute. Embodiments of the present invention are not limited to the illustrated arrangement. Those of ordinary skill in the art will recognize that other configurations and modes of analyte transport are possible.

Analytes and other solutes or molecules can passively diffuse through skin to the skin surface. As one example, alcohol appears at the skin surface approximately 30 minutes after oral ingestion. Solutes can diffuse through skin, into and/or through sweat from wounds, cuts, or other pathways through skin. Diffusion is faster with a higher concentration gradient (i.e., greater change in concentration of the analyte over shorter distance it has to travel by diffusion). With further reference to Fig. 6, device 600 senses diffusing analytes. In this embodiment, the electrode 628 is removed and the gel 615 furthest away from the skin 12 would initially have low or zero concentration of the analyte or analytes to be sensed by porous sensor 602. For solutes in fluids such as water, oils, glycols, or other fluids or liquids, the porous sensor 602 could be filled with a fluid or gel to create a diffusion pathway through porous sensor 602. The gel 615 could be a hydrogel or a gel containing a liquid that increases skin permeability such as, for example, glycol. Advantages of using a porous sensor 602 according to an embodiment of the present invention in a device that senses diffusing analytes may include the following. Firstly, in an embodiment including a lateral flow or other flow technique across sensors (e.g., as shown in Figs. 1B or 2B), the diffusion pathway could be long (e.g., length of several mm or more), which reduces the concentration gradient of the analyte to be sensed. Secondly, the gel 615 furthest away from skin 12 could have a large volume (e.g., several mm thick) such that over time the gel 615 will maintain a low concentration of the analyte or analytes to be sensed by porous sensor 602. In this manner, gel 615 acts as a material into which an analyte can diffuse with a lesser concentration of the analyte than the analyte concentration found in skin. In an embodiment where the analyte is volatile (e.g., alcohol), the gel 615 furthest away from the skin 12 could be removed such that ambient air would represent the low concentration material to enable a direction of diffusion from skin to air. Thirdly, the device may further include advantageous features of the present invention described above, such as bringing analytes closer to the sensing surface, which is important for sensing of very low concentration analytes. Those of ordinary skill in the art will recognize how to modify or configure the devices according to embodiments of the present invention so as to effectively operate in other applications.

The following examples are provided to help illustrate the present invention, and are not comprehensive or limiting in any manner.
EXAMPLE 1 - In one embodiment, a method of making a sensing device, such as a sensing device similar to device 300a, includes coating a first and a second polyimide track etch membrane with an electrode. The membrane may be, for example, one such as those manufactured by AR-Brown or IT4IP. The membranes have an initial thickness of 7.6, 13, or 25 µm, pore sizes of 100 nm, and a pore density of at least on average of one pore for every linear 5 µm. The coating may be a 10 nm coating of gold electrode. Adhesive spacer balls are then spray coated onto the gold coated side of the first prepared membranes. The first membrane is then dried of the solution used for the spray coating. The adhesive spacer balls may be from Sekisui Micropearl and have a diameter of about 5 µm, for example. The first and second prepared membranes are then adhered together with the gold-coated surfaces facing each other. To accomplish this, the first and second membranes may be hot-laminated at 140 °C, which activates the adhesive. One of several types of chemical or bio sensors can then be created through solution or chemical treatment of the gold surfaces. The treatments may typically be performed using a vacuum-degassing step to ensure solution treatment penetrates into the sandwiched structure of the two substrates. Lastly, a dilute solution of a water soluble polymer, such as Polyvinylpyrrolidone (PVP), is used to coat all surfaces of the film, again using vacuum for degassing, to create a sensing device which is hydrophilic (i.e., sweat wets through the device with no pressure required). The PVP coating process has been shown to be commercially viable for track-etch membranes down to even 10 nm pore sizes. If needed, one or more external surfaces of the resulting device can be cleaned of PVP if adhesion to another material or surface needs to be improved.
EXAMPLE 2 - In one embodiment, a sensing device includes two sheets of porous carbon paper, like those utilized in fuel cells, laminated on both sides of an ultrathin (e.g., 10 µm thick) sheet of porous paper or polymeric material. Other options include spacing materials used as spacers in electrolytic capacitors. The sensor is utilized for sensing chemistries and modalities known to work effectively with carbon electrodes.
EXAMPLE 3 - In one embodiment, a device of the present invention is created using aligned photolithographic methods. Using such a method would provide more homogenous electrical impedance and flow through the device.

## Claims

1. An electronic analyte sensor for sensing an analyte in sweat comprising at least one porous electrode providing an electrical response to the presence of an analyte, the at least one porous electrode comprising at least two porous substrates (310, 312, 550, 552, 554, 556) each having an electrically conductive surface, said porous substrates having a generally planar surface, wherein the analyte flow path is perpendicular to said planar surface and through at least one of said porous substrates (310, 312, 550, 552, 554, 556), **characterized in that** the sensor includes a porous spacer layer (315, 560) defining a gap between said porous substrates (310, 312, 550, 552, 554, 556), and **in that** at least one of said conductive surfaces provides the electrical response to the presence of said analyte in the analyte flow.

2. The electronic analyte sensor of claim 1, wherein said generally planar surfaces of said porous substrates (310, 312, 550, 552, 554, 556) are adapted to be positioned on skin generally coplanar with said skin and said analyte flow path is from skin.

3. The electronic analyte sensor of claim 2, wherein an analyte flow of sweat is vertically confined in said analyte flow path above sweat ducts in said skin.

4. The electronic analyte sensor of claim 2, wherein said electric response is a change in charge transfer with said electrically conductive surface.

5. The electronic analyte sensor of claim 1 wherein said at least two porous substrates (310, 312, 550, 552, 554, 556) are spaced apart by 10 µm or 5 µm.

6. The electronic analyte sensor of claim 1, wherein said generally planar surfaces are adapted to be positioned on skin generally coplanar with said skin; wherein the electronic analyte sensor further comprises at least one iontophoresis electrode configured to cause an analyte flow.

7. An electronic analyte sensor of claim 1, wherein generally planar surfaces are adapted to be positioned on skin generally coplanar with said skin; and wherein the electronic analyte sensor further comprises at least a first material (532) for collecting an analyte diffusing through said skin, said first material having a lower concentration of said analyte than a concentration of said analyte in said skin, said at least one porous substrate being positioned between said skin and said first material (532);.

8. The electronic analyte sensor of claim 7, wherein said first material (532) includes a liquid or a hydrogel.

9. The electronic analyte sensor of claim 7, further comprising:
a second material (615) being positioned between said skin and said porous substrate when said sensor is placed on said skin, said second material (615) including a component that increases skin permeability, said component being transferable to said skin when said sensor is placed on said skin.

## Patentansprüche

1. Elektronischer Analytsensor zum Erfassen eines Analyten in Schweiß, umfassend mindestens eine poröse Elektrode, die eine elektrische Antwort auf die Anwesenheit eines Analyten liefert, wobei die mindestens eine poröse Elektrode mindestens zwei poröse Substrate (310, 312, 550, 552, 554, 556) umfasst, die jeweils eine elektrisch leitende Oberfläche aufweisen, wobei die porösen Substrate eine im Allgemeinen ebene Oberfläche aufweisen, wobei der Analytfließpfad senkrecht zu der ebenen Oberfläche und durch mindestens eines der porösen Substrate (310, 312, 550, 552, 554, 556) verläuft, **dadurch gekennzeichnet, dass** der Sensor eine poröse Abstandsschicht (315, 560) enthält, die einen Spalt zwischen den porösen Substraten (310, 312, 550, 552, 554, 556) definiert, und dass mindestens eine der leitenden Oberflächen die elektrische Antwort auf das Vorhandensein des Analyten in dem Analytfluss liefert.

2. Elektronischer Analytsensor nach Anspruch 1, wobei die im Allgemeinen ebenen Oberflächen der porösen Substrate (310, 312, 550, 552, 554, 556) angepasst sind, um auf einer Haut positioniert zu werden, die im Allgemeinen koplanar mit der Haut ist, und der Analytfließpfad von einer Haut stammt.

3. Elektronischer Analytsensor nach Anspruch 2, wobei ein Analytschweißfluss in dem Analytfließpfad über Schweißkanäle in der Haut vertikal begrenzt ist.

4. Elektronischer Analytsensor nach Anspruch 2, wobei die elektrische Antwort eine Änderung einer Ladungsübertragung mit der elektrisch leitenden Oberfläche ist.

5. Elektronischer Analytsensor nach Anspruch 1, wobei die mindestens zwei porösen Substrate (310, 312, 550, 552, 554, 556) um 10 µm oder 5 µm voneinander beabstandet sind.

6. Elektronischer Analytsensor nach Anspruch 1, wobei die im Allgemeinen ebenen Oberflächen angepasst sind, um im Allgemeinen koplanar mit der Haut auf der Haut positioniert zu werden;
wobei der elektronische Analytsensor ferner mindestens eine lontophoreseelektrode umfasst, die konfiguriert ist, um einen Analytfluss zu verursachen.

7. Elektronischer Analytsensor nach Anspruch 1, wobei die im Allgemeinen ebenen Oberflächen angepasst sind, um im Allgemeinen koplanar mit der Haut auf der Haut positioniert zu werden; und
wobei der elektronische Analytsensor ferner mindestens ein erstes Material (532) zum Sammeln eines durch die Haut diffundierenden Analyten umfasst, wobei das erste Material eine niedrigere Konzentration des Analyten als eine Konzentration des Analyten in der Haut aufweist, wobei das mindestens eine poröse Substrat zwischen der Haut und dem ersten Material (532) positioniert ist;.

8. Elektronischer Analytsensor nach Anspruch 7, wobei das erste Material (532) eine Flüssigkeit oder ein Hydrogel enthält.

9. Elektronischer Analytsensor nach Anspruch 7, ferner umfassend:
ein zweites Material (615), das zwischen der Haut und dem porösen Substrat positioniert ist, wenn der Sensor auf der Haut angeordnet ist, wobei das zweite Material (615) eine Komponente enthält, die eine Hautpermeabilität erhöht, wobei die Komponente auf die Haut übertragbar ist, wenn der Sensor auf der Haut angeordnet ist.

## Revendications

1. Capteur électronique d'analyte servant à détecter un analyte dans de la sueur, comprenant au moins une électrode poreuse produisant une réponse électrique à la présence d'un analyte, l'au moins une électrode poreuse comprenant au moins deux substrats poreux (310, 312, 550, 552, 554, 556) présentant chacun une surface électriquement conductrice, lesdits substrats poreux présentant une surface généralement plane, le trajet d'écoulement d'analyte étant perpendiculaire à ladite surface plane et traversant au moins l'un desdits substrats poreux (310, 312, 550, 552, 554, 556), **caractérisé en ce que** le capteur comporte une couche d'espacement poreuse (315, 560) définissant un espace entre lesdits substrats poreux (310, 312, 550, 552, 554, 556), et **en ce qu'**au moins l'une desdites surfaces conductrices produit la réponse électrique à la présence dudit analyte dans l'écoulement d'analyte.

2. Capteur électronique d'analyte selon la revendication 1, dans lequel lesdites surfaces généralement planes desdits substrats poreux (310, 312, 550, 552, 554, 556) sont conçues pour être positionnées sur une peau généralement coplanaire avec ladite peau, et ledit trajet d'écoulement d'analyte provient de la peau.

3. Capteur électronique d'analyte selon la revendication 2, dans lequel un écoulement d'analyte de sueur est confiné verticalement dans ledit trajet d'écoulement d'analyte au-dessus des canaux sudoripares de ladite peau.

4. Capteur électronique d'analyte selon la revendication 2, dans lequel ladite réponse électrique est un changement dans le transfert de charge avec ladite surface électriquement conductrice.

5. Capteur électronique d'analyte selon la revendication 1, dans lequel lesdits au moins deux substrats poreux (310, 312, 550, 552, 554, 556) sont espacés de 10 ou 5 µm.

6. Capteur électronique d'analyte selon la revendication 1, dans lequel lesdites surfaces généralement planes sont conçues pour être positionnées sur une peau généralement coplanaire avec ladite peau ;
le capteur électronique d'analyte comprenant en outre au moins une électrode d'iontophorèse configurée pour provoquer un écoulement d'analyte.

7. Capteur électronique d'analyte selon la revendication 1, dans lequel les surfaces généralement planes sont conçues pour être positionnées sur une peau généralement coplanaire avec ladite peau ; et
le capteur électronique d'analyte comprenant en outre au moins un premier matériau (532) permettant de recueillir un analyte se diffusant à travers ladite peau, ledit premier matériau présentant une concentration dudit analyte inférieure à une concentration dudit analyte dans ladite peau, ledit au moins un substrat poreux étant positionné entre ladite peau et ledit premier matériau (532).

8. Capteur électronique d'analyte selon la revendication 7, dans lequel ledit premier matériau (532) comporte un liquide ou un hydrogel.

9. Capteur électronique d'analyte selon la revendication 7, comprenant en outre :
un second matériau (615) positionné entre ladite peau et ledit substrat poreux lorsque ledit capteur est placé sur ladite peau, ledit second matériau (615) comportant un composant qui augmente la perméabilité de la peau, ledit composant pouvant être transféré sur ladite peau lorsque ledit capteur est placé sur ladite peau.
